# FASCICULE DE BREVET EUROPEEN

(11) **EP 0 642 804 B1**
(45) Date de publication et mention de la délivrance du brevet: **06.09.2000**
(21) Numéro de dépôt: 94401546.0
(22) Date de dépôt: 05.07.1994
(51) Int. Cl.: A61M 25/02

(54) **Dispositif de protection et de maintien notamment pour sonde médicale ou drain**
Vorrichtung zum Schutz und zur Halterung, hauptsächlich für eine medizinische Sonde oder Drain
Device for protecting and holding, particularly for a medical probe or drain

(30) Priorité: 05.07.1993 FR 9308206; 14.02.1994 FR 9401649
(43) Date de publication de la demande: 15.03.1995
(73) Titulaire: ASSISTANCE PUBLIQUE-HOPITAUX DE PARIS, 75100 Paris (FR)
(72) Inventeur: Similowski, Thomas, F-92130 Issy-Les-Moulineaux (FR)
(74) Mandataire: Phélip, Bruno

(56) Documents cités:
- EP-A- 0 567 029
- FR-A- 2 371 934
- US-A- 3 783 876
- US-A- 4 397 647
- US-A- 5 105 807
- US-A- 5 123 410
- US-A- 5 267 970

## Description

L'invention concerne un dispositif de protection et de maintien de moyens destinés à mettre l'intérieur du corps d'un patient en communication avec l'extérieur.

Dans le domaine médical, il est en effet couramment nécessaire de mettre en communication l'intérieur du corps d'un patient avec un dispositif adapté aux soins à apporter, par exemple un système d'aspiration ou un système de ventilation artificielle.

Un système d'aspiration est notamment utilisé pour effectuer un drainage thoracique ou abdominal. Il s'agit d'une méthode fréquemment appliquée en réanimation et en chirurgie. Elle consiste à mettre en communication l'intérieur du thorax ou de l'abdomen avec le système d'aspiration, en général pour évacuer un épanchement (gazeux ou liquidien) de la cavité pleurale ou abdominale, drainer un site opératoire ou un processus pathologique.

De façon classique, la fixation du drain est assurée par une suture à la peau. Le drain fixé est recouvert d'un pansement constitué de compresses et d'adhésifs.

La fixation d'un tel drain pose de nombreux problèmes.

On constate tout d'abord qu'un drain se plie souvent à proximité de son orifice de sortie, ce qui compromet l'efficacité des soins apportés aux patients. De plus, la réalisation des pansements est relativement difficile et longue. Enfin, la présence de ce pansement rend difficile l'accès au drain pour vérifier sa position et sa bonne perméabilité.

D'autres moyens de mise en communication de l'intérieur du corps d'un patient avec l'extérieur sont constitués par les sondes , notamment utilisées dans les unités de réanimation , en anesthésie au bloc opératoire et au réveil , ainsi que lors des interventions et transports médicalisés urgents .

On sait que les sondes d'intubation trachéales permettent d'assurer la ventilation artificielle de patients et la protection de leurs voies aériennes.

Ces sondes peuvent être introduites par voie buccale (intubation oro-trachéale) ou par voie nasale (intubation naso-trachéale).

Leur utilisation pose certains problèmes , touchant à leur protection et à leur fixation .

Dans le cas de la voie oro-trachéale, le patient risque de mordre la sonde qui n'est pas protégée et par là, de compromettre sa ventilation.

Le maintien de ces sondes est également un impératif majeur. En effet, elles ont tendance à descendre dans les voies aériennes. Un maintien inadéquat peut déboucher sur une malposition distale de la sonde qui de trachéale devient bronchique ( "intubation sélective" ) , compromettant la ventilation artificielle , ou sur la sortie de la sonde de la trachée ( "extubation" ) , pouvant constituer une menace vitale. Un maintien inadéquat peut également, par les mouvements répétés imposés à la sonde lors des multiples sollicitations externes, être source de traumatismes. Pour des raisons anatomiques, la fixation correcte des sondes d'intubation est plus difficile à assurer par la voie oro-trachéale que par la voie naso-trachéale .

Ainsi, les sondes destinées à la ventilation artificielle sont généralement fixées par l'intermédiaire d'un cordon qui est placé autour de la tête du patient. Pour assurer une fixation correcte, il est nécessaire de serrer fortement ce cordon , ce qui est peu confortable et source de lésions parfois sérieuses ( escarres aux points de friction ...). Cette nécessité de serrage rend la libération de la sonde , en vue d'une mobilisation, longue et délicate .

On note en effet que les sondes d'intubation trachéales doivent parfois être mobilisées , vers l'avant ou vers l'arrière , pour en optimiser la position . C'est le cas dans les suites immédiates de leur mise en place, après une intervention médicale spécifique tendant à les déplacer (endoscopie bronchique ) ou une sollicitation accidentelle , ou lorsque le contrôle systématique de leur position montre un déplacement .

Pour limiter le risque d'infections pulmonaires , le personnel paramédical doit , chez les patients ventilés artificiellement , effectuer plusieurs fois par jour des soins de bouche . En particulier lors de l'intubation oro-trachéale, la qualité de ces soins implique la libération de la sonde de tout dispositif de protection ou de fixation. Le système du cordon , passé directement autour de la sonde et serré , doit donc être retiré puis remis en place à chaque soin et à chaque mobilisation de la sonde , ce qui est une source importante de difficultés, de perte de qualité de soin et de temps .

Les sondes gastriques sont , quant à elles , destinées à l'alimentation des patients ou à la réalisation d'une aspiration digestive . Celles-ci sont presque constamment introduites par voie nasale ( d'où leur dénomination commune de sondes naso-gastriques ). Le diamètre des sondes gastriques est faible , très inférieur ( de 4 à 10 fois ) à celui des sondes d'intubation trachéale ; leur débattement est grand . Elles ont tendance à se déplacer , en général vers l'extérieur du patient , sous l'effet de leur propre poids et des tractions exercées par les dispositifs d'alimentation ou d'aspiration auxquels elles sont reliées . Elles peuvent également se déplacer très aisément sous l'effet de sollicitations accidentelles ou volontaires, du patient ou du personnel soignant .

Actuellement, les sondes naso-gastriques sont généralement maintenues par des systèmes adhésifs . Du fait de leur faible diamètre , il n'est en général pas possible d'en assurer la fixation grâce à des cordons, car ceux-ci ne peuvent pas y être suffisamment serrés .

Elles posent le même type de problème que les sondes pour ventilation artificielle, en ce qui concerne les manipulations qu'il est nécessaire d'effectuer pour réaliser une opération sur la sonde, telle que son déplacement.

Pour résoudre ces problèmes, il a déjà été proposé plusieurs dispositifs.

On peut notamment citer des systèmes comprenant des mâchoires rigides , entre lesquelles la sonde peut être fixée. Ces mâchoires sont maintenues à la tête du patient par l'intermédiaire d'un harnais ou de liens .

Ces systèmes permettent de fixer solidement les sondes introduites par voie buccale ou nasale , sans utiliser de rubans adhésifs, généralement fixés sur le visage du patient . Ce système évite également les risques de pincement de la sonde et réduit ses mouvements , ce qui atténue les risques de traumatisme de la trachée et du larynx.

Cependant , ces systèmes sont relativement complexes, coûteux et inconfortables pour les patients , de par leur poids et le volume qu'ils occupent au niveau du massif facial. De plus, ils ne sont pas d'une utilisation facile. En effet, la fixation de la sonde aux mâchoires nécessite la pose de rubans adhésifs ou d'épingles de sécurité.

On connaît les documents FR-2 371 934 (qui correspond au préambule de la revendication 1), US-5,123,410 et US-3,783,876 qui décrivent des systèmes ayant pour seul objet la fixation d'une sonde et non sa protection. De surcroît, ces systèmes ne peuvent pas être directement mis en place sur la sonde. Celle-ci doit être introduite à travers le système. Ceci présente des inconvénients puisque la mise en place d'une sonde est difficile, voire impossible pour celles qui comportent un système externe pour la surveillance de l'état de gonflage du ballonet d'étanchéité. De plus, l'introduction de la sonde à travers le dispositif impose de la déconnecter du dispositif, par exemple de ventilation, auquel elle relie le patient. Ces systèmes ne peuvent donc pas être utilisés sans rupture de la continuité des soins et sans risque pour la sécurité du patient.

On peut également citer les documents EP-0 567 029 et US-4,397,647 qui concernent la fixation de cathéters à la surface de la peau. Du fait de leur encombrement, les dispositifs décrits ne peuvent pas être utilisés pour tous types de moyens de mise en communication de l'intérieur du corps d'un patient avec l'extérieur. En effet, ces dispositifs ne peuvent pas être placés, même partiellement, à l'intérieur d'une cavité et ils ne peuvent donc pas convenir à des sondes d'intubation.

Ils présentent de plus un inconvénient majeur puisqu'une fois en place sur le cathéter, ils le plie ce qui doit absolument être évité pour des sondes ou des drains.

Enfin, le brevet US-5,105,807 décrit un dispositif pour la fixation de sondes nasales qui comprend un élément tubulaire dont la paroi est en permanence partiellement ouverte pour permettre l'introduction de la sonde et une couche de mousse compressible placée en contact sur l'élément tubulaire, sur sa paroi extérieure, pour bloquer l'ensemble à l'intérieur d'une narine. La sonde est bloquée à l'intérieur de l'élément tubulaire au moyen de systèmes additionnels de verrouillage.

Ce dispositif est, par sa conception, limité à des sondes de petit diamètre, les dimensions de l'ouverture prévue étant nécessairement limitées pour assurer la fixation de la sonde. De plus, le dispositif ne recouvre pas la sonde et celle-ci n'est pas totalement protégée. Enfin, il faut prévoir des systèmes additionnels de verrouillage qui risquent de détériorer la sonde et qui limitent également son diamètre.

L'invention a pour objet un dispositif de protection et de maintien de moyens destinés à mettre l'intérieur du corps d'un patient en communication avec l'extérieur, permettant de pallier ces inconvénients tout en étant d'une fabrication peu coûteuse et d'une utilisation simple, ce dispositif étant, de plus, parfaitement adaptable à des moyens de dimensions quelconques.

Ce dispositif peut être utilisé pour des drains de toute nature et de toute localisation ainsi que pour des sondes d'intubation trachéales mises en place par voie orale ou nasale et également pour d'autres types de sonde, dont les sondes gastriques destinées à l'alimentation ou à l'aspiration digestive .

Selon l'invention, le dispositif de protection et de maintien est defini dans la revendication 1.

La partie des moyens de mise en communication qui se trouve à l'intérieur du dispositif est ainsi maintenue et protégée contre tout risque de déformation.

Dans la position de fermeture, lesdits éléments recouvrent totalement la partie desdits moyens de mise en communication se trouvant à l'interieur de la cavité et peuvent ainsi assurer à eux seuls leur maintien dans ledit dispositif et dans la position d'ouverture, lesdits éléments sont écartés l'un de l'autre pour ouvrir la cavité latéralement.

De façon préférée, le dispositif comprend des moyens de fixation sur ledit corps.

Ils permettent d'éviter tout déplacement non souhaité des moyens de mise en communication.

De façon préférée, le dispositif est constitué de deux éléments sensiblement semi-cylindriques.

Dans un mode de réalisation, lesdits éléments sont séparables.

Le dispositif comprend alors des moyens permettant le coulissement d'un élément dans l'autre.

De façon préférée, la paroi interne de la cavité est recouverte d'un matériau élastique et anti-dérapant.

De façon à adapter le dispositif à des moyens de mise en communication de dimensions quelconques, au moins une pièce en un matériau rigide est placée sur la paroi interne de la cavité.

Le dispositif comprend au moins une pièce en saillie, fixée auxdits éléments.

Dans une première variante, ladite pièce en saillie est réalisée en un matériau rigide.

Dans ce cas, la pièce en saillie est notamment destinée à supporter les moyens de fixation et le dispositif est plus particulièrement conçu pour les sondes.

Dans une deuxième variante, ladite pièce en saillie comprend une pièce réalisée en une matière absorbante, de préférence fixée à une partie rigide. Dans ce cas, les moyens de fixation peuvent être constitués par une zone adhésive placée sur la face arrière. Le dispositif est alors plus particulièrement conçu pour des drains.

De préférence, les parties au contact du corps sont recouvertes d'un matériau anti-escarre.

Enfin, le dispositif selon l'invention peut être utilisé en tant que dispositif à usage unique.

L'invention sera mieux comprise et d'autres buts, avantages et caractéristiques de celle-ci apparaîtront plus clairement à la lecture de la description qui suit, faite en référence aux dessins annexés, sur lesquels :
- la figure 1 représente une vue en perspective d'un premier dispositif conforme à l'invention, mis en place autour d'une sonde d'intubation ,
- la figure 2 représente une vue selon II de la figure 1,
- la figure 3 représente le dispositif de la figure 1, lors de sa mise en place autour de la sonde et,
- la figure 4 représente une vue partielle de face d'un dispositif, en position fermée et,
- la figure 5 comprend les figures 5a et 5b qui représentent chacune un élément constitutif d'un autre dispositif selon l'invention, destiné à un drain.

Les éléments communs aux différentes figures seront désignés par les mêmes références.

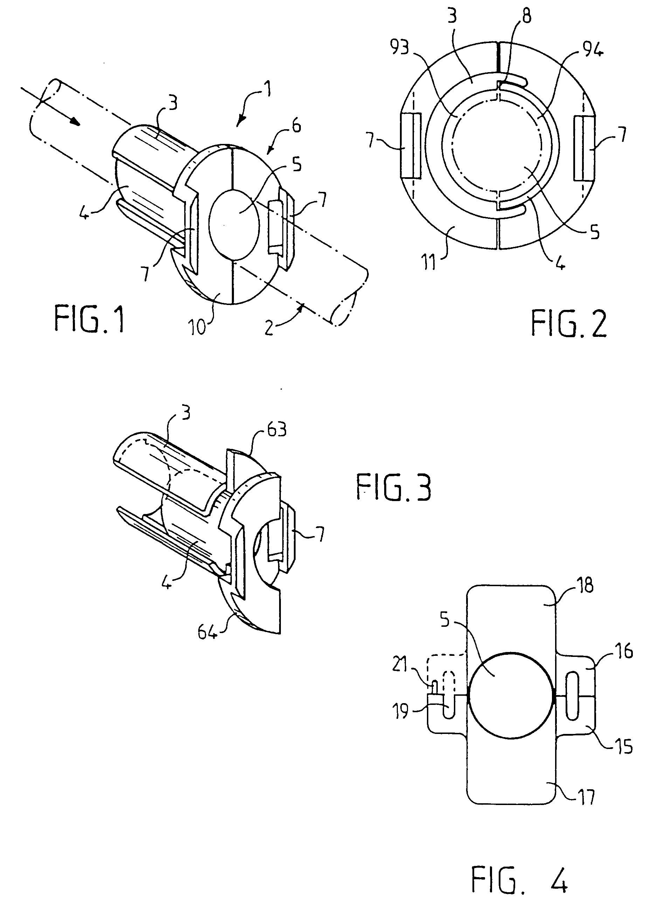

Sur la figure 1, la référence 1 désigne un dispositif selon l'invention qui est mis en place autour d'une sonde d'intubation 2. Le dispositif est alors en position de fermeture.

Le dispositif 1 comprend essentiellement deux éléments séparables 3 et 4, sensiblement semi-cylindriques qui, lorsqu'ils sont rendus solidaires, définissent une cavité 5 dont le diamètre intérieur correspond sensiblement au diamètre extérieur de la sonde 2. Comme on le verra ultérieurement, des moyens sont prévus pour améliorer le maintien de la sonde 2 dans la cavité 5.

Ainsi, dans l'exemple représenté, les éléments 3 et 4 sont coaxiaux et définissent une cavité tubulaire cylindrique, mais cela ne représente qu'une forme de réalisation.

Dans cette position de fermeture, le dispositif recouvre totalement la partie de la sonde se trouvant à l'interieur de la cavité. En particulier, la paroi tubulaire du dispositif est fermée et ne comporte aucune ouverture pouvant permettre le passage de la sonde. La mise en place du dispositif sur la sonde ne peut être réalisée que si le dispositif est en position ouverte, position qui sera notamment décrite au regard de la figure 3.

Le dispositif 1 comporte également une paroi annulaire 6 en saillie qui supporte des moyens 7 permettant la fixation d'un système de maintien du dispositif 1 sur le patient, le système de maintien n'est pas représenté sur les figures.

Ces moyens 7 sont essentiellement constitués par des moyens de retenue permettant d'attacher un cordon, qui constitue un système de maintien classique, de part et d'autre de la cavité 5. Les moyens 7 sont, dans l'exemple représenté sur la figure 1, en saillie sur la face extérieure 10 de l'anneau 6.

D'autres moyens de fixation pourraient être prévus. Ils peuvent également être fixés directement sur les éléments 3 et/ou 4.

On se réfère maintenant à la figure 3 qui illustre un dispositif selon l' invention lors de sa mise en place sur la sonde 2 (non représentée sur la figure 3). Cette figure 3 montre que la paroi annulaire 6 est composée de deux éléments, 63 et 64, chacun d'eux étant porté par un élément constitutif du dispositif, 3 ou 4.

Il est aisé de comprendre comment s'effectue la mise en place du dispositif autour de la sonde d'intubation 2. Le dispositif est en position d'ouverture, les éléments 3 et 4 étant écartés l'un de l'autre, de façon à ouvrir la cavité 5. Celle-ci est alors accessible. On place tout d'abord la sonde dans l'un des deux éléments 3 ou 4, par exemple l'élément 3, puis on fait coulisser l'élément 4 dans l'élément 3, comme le montre la figure 3. Il n'est pas nécessaire de débrancher la sonde du dispositif auquel elle est reliée pour effectuer cette opération. Lorsque l'élément 4 est complètement enfoncé dans l'élément 3, le dispositif 1 est en place autour de la sonde 2, comme l'illustre la figure 1.

En référence à la figure 2, le coulissement d'un élément par rapport à l'autre est rendu possible grâce à la rainure 8 que supporte l'élément 3 et dans laquelle peuvent s'engager les bords extérieurs de l'élément 4. D'autres systèmes pourraient également être prévus pour rendre les éléments 3 et 4 solidaires.

Le dispositif ayant pour objet de maintenir la sonde, on peut prévoir des moyens, à l'intérieur de la cavité 5, notamment constitués par une pellicule de mousse ou de tout autre matériau élastique et anti-dérapant , pouvant épouser la forme de la sonde et combler les jeux éventuels, de faible importance, qui peuvent exister entre la cavité 5 et la sonde 2. Ces moyens ne sont pas illustrés sur les différentes figures.

Une fois que le dispositif 1 est mis en place autour de la sonde 2, il est solidaire de cette dernière et la stoppe. Ainsi, les éléments 3 et 4 peuvent assurer directement le maintien de la sonde et le dispositif selon l'invention permet d'éviter la présence de systèmes de verrouillage additionnels. On attache alors le dispositif 1 au patient par un système de maintien approprié, tel qu'un cordon, dont les deux extrémités ont été préalablement attachées aux moyens 7 de fixation.

Ce cordon est classiquement attaché autour de la tête du patient. Grâce au dispositif 1, il n'est pas nécessaire de serrer très fortement le cordon autour de la tête du patient, puisque c'est le dispositif 1 qui assure le maintien de la sonde 2 et non pas directement le cordon.

De plus, lorsque la sonde 2 est une sonde oro-trachéale, la présence du dispositif 1 évite que le patient ne morde la sonde. Le passage de gaz dans la sonde ne peut donc pas être perturbé.

Enfin, un des avantages essentiels du dispositif pour sonde selon l'invention est qu'il peut être facilement retiré de la sonde, sans qu'il soit nécessaire, au préalable, de retirer le système de maintien (cordon). En effet, lorsqu'il est nécessaire d'agir sur la sonde, par exemple pour la repositionner ou effectuer des soins de bouche, il suffit de séparer l'un de l'autre les deux éléments constitutifs 3 et 4 du dispositif 1 en les faisant coulisser en sens inverse de celui de la mise en place. Le retrait du dispositif 1 s'effectue très facilement. Lorsque les soins de bouche sont terminés, on remet en place le dispositif 1 autour de la sonde 2, comme cela a été expliqué précédemment.

Ce retrait et cette mise en place n'obligent pas à détacher le cordon du dispositif 1. Celui-ci est simplement attaché autour de la tête du patient, après mise en place du dispositif 1 autour de la sonde 2. Il n'est pas non plus nécessaire de déconnecter la sonde du dispositif, notamment du ventilateur, auquel elle est reliée. Par conséquent , le dispositif 1 facilite les soins d'autant plus qu'il n'impose jamais de compromettre la sécurité du patient .

Ainsi, le dispositif 1 supprime de nombreuses opérations qui étaient nécessaires avec les techniques précédentes et assure donc un gain de temps important au personnel paramédical, ainsi qu'une amélioration de la qualité des soins prodigués et le confort du patient.

On sait que les diamètres des sondes peuvent varier de façon importante. Ils sont classiquement compris entre 2 et 11 millimètres. C'est pourquoi, en fonction de la sonde utilisée, on peut placer, sur la paroi interne de chacun des éléments constitutifs 3 et 4, des pièces 93 et 94 qui épousent la forme de la paroi interne de ces éléments. Elles sont, de préférence, réalisées en une matière rigide et permettent de diminuer le diamètre interne de la cavité 5.

Ainsi, le dispositif selon l'invention est parfaitement adaptable à des sondes de diamètre quelconque.

De plus, les éléments 3 et 4 ne définissent pas nécessairement une cavité cylindrique. En effet, grâce à la présence des moyens en un matériau élastique et non dérapant et/ou des pièces 93 et 94, on peut adapter la forme de la cavité définie par les éléments à celle de la sonde.

Le dispositif selon la figure 1 peut également être utilisé pour une sonde naso-trachéale. Les moyens de fixation du système de maintien peuvent être adaptés à cette application particulière. En effet, dans cette application, la face extérieure 10 de la paroi annulaire 6 est dirigée vers le patient et il est donc préférable que cette face ne présente pas d'élément en saillie, tels que les moyens d'attache 7. Ceux-ci peuvent donc être déplacés et placés sur la face interne 11 de la bague 6. Cette disposition n'est pas représentée sur les figures.

Dans cette application, la face extérieure 10 de la paroi annulaire 6, dirigée vers le patient , peut être recouverte de tout type de matériau protecteur , destiné à limiter le risque d'escarre du nez par frottement . Cette variante s'applique à la fixation des sondes d'intubation mises en place par voie nasale, mais aussi particulièrement à la fixation des sondes naso-gastriques.

Lorsque le dispositif selon l'invention est utilisé pour une sonde d'intubation naso-trachéale, il permet essentiellement de résoudre les problèmes liés à la fixation de la sonde.

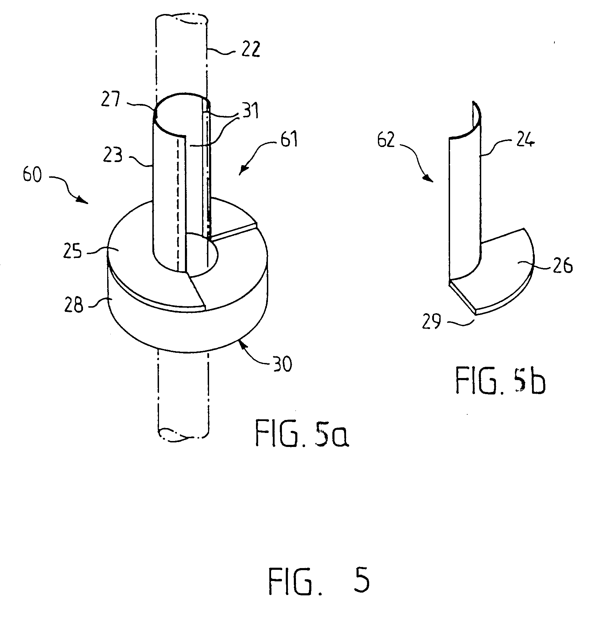

En référence à la figure 4, un autre mode de réalisation du dispositif selon l'invention est représenté. Ce dispositif 20 comprend essentiellement deux éléments 12 et 13 semi-cylindriques qui sont reliés entre eux par une articulation 14. Chaque élément supporte des moyens 15, 16 destinés à la fixation du système de maintien et qui sont placés par exemple sur une plaque 17, 18 en saillie sur les éléments 12 et 13 . Les moyens de fixation pourraient être prévus directement sur les éléments 12 et 13.

Dans cet exemple, les éléments 12 et 13 sont également coaxiaux et définissent une cavité tubulaire cylindrique.

Le dispositif 20 est mis en place de la façon suivante. Les deux éléments 12 et 13 étant écartés l'un de l'autre, on place tout d'abord la sonde dans un des deux éléments. La paroi tubulaire du dispositif ne présente pas d'ouverture par laquelle la sonde pourrait passer et, avec les éléments 13 et 14, le dispositif peut assurer directement le maintien de la sonde.

Comme cela a été expliqué pour le dispositif décrit en référence aux figures 1 à 3, le dispositif 20 comprend, sur la face interne de chacun des éléments 12 et 13, des moyens tels qu'une mousse qui permet au dispositif 20 d'être maintenu fermement sur la sonde d'intubation.

On peut également prévoir, comme précédemment, des éléments demi-cylindriques qui épousent la paroi interne des éléments 12 et 13 pour adapter le diamètre interne de la cavité du dispositif 20 à la sonde d'intubation utilisée.

En référence maintenant à la figure 5, le dispositif selon l'invention est mis en place autour d'un drain 22, par exemple thoracique ou abdominal.

Le dispositif 60 est constitué essentiellement de deux pièces 61 et 62, la pièce 61 est représentée à la figure 5a et la pièce 62 à la figure 5b.

Comme dans les exemples de réalisation décrits précédemment, chaque pièce 61, respectivement 62 comprend un élément 23, respectivement 24. Sur la figure 5, les éléments 23 et 24 sont sensiblement cylindriques. Les pièces 61 et 62 peuvent être écartées l'une de l'autre et rendues solidaires par des moyens tels que ceux décrits précédemment, c'est-à-dire des moyens permettant le coulissement des éléments 23 et 24 l'un dans l'autre. C'est ce mode de réalisation qui est illustré sur la figure 5. L'élément 23 comporte des rainures 31 dans lesquelles peut s'engager l'élément 24.

Dans l'exemple représenté, les éléments 23 et 24 sont coaxiaux mais ceci n'est qu'une forme de réalisation possible.

Le dispositif 60 est mis en place autour du drain 22 en faisant coulisser les éléments 23 et 24 l'un dans l'autre.

Ils définissent une cavité 27 tubulaire et cylindrique pour le passage et la protection du drain 22. La paroi tubulaire du dispositif ne comporte pas d'ouverture par laquelle la sonde pourrait passer et les éléments 23 et 24 peuvent assurer à eux seuls le maintien du drain. La présence des deux éléments 23 et 24 permet d'éviter que le drain 22 ne se plie, ce qui élimine les risques entrainés par les drains classiques.

Les éléments 23 et 24 peuvent être réalisés de longueur légèrement différente. Ainsi, la courbure naturelle d'un drain selon la gravité peut être respectée, sans que le dispositif ne provoque de pliure.

De façon préférée, le dispositif 60 comporte une pièce en saillie 28, réalisée en un matériau absorbant, de préférence hémostatique. Cette pièce 28 peut être réalisée d'un seul tenant. Elle est alors fixée à l'un des éléments 23 ou 24. Sur le mode de réalisation illustré à la figure 6a, la pièce 28 est fixée à l'élément 23. Elle peut également être réalisée en deux parties, chacune d'elles étant fixée à l'un des éléments 23 ou 24.

Pour faciliter la fixation de la pièce 28 sur le dispositif 60, celui-ci peut comporter une partie rigide, également en saillie 25, 26.

La fonction de cette pièce 28 est d'absorber les suffusions au pourtour de l'orifice ménagé dans le corps du patient et dans lequel s'insère le drain 22. Cette pièce 28 remplace donc la partie "compresse" du pansement traditionnel et elle simplifie considérablement la réalisation de ce dernier.

Le dispositif 60 peut être classiquement fixé au patient, par une suture à la peau. De façon préférée, une zone adhésive est prévue sur la face arrière 29 des parties en saillie 25 ou 26 ou encore sur la face arrière 30 de la pièce 28, lorsque le dispositif 60 comporte une telle pièce, les faces arrières étant destinées à venir en contact avec la peau du patient.

Comme cela a été indiqué pour les autres modes de réalisation, la cavité 27 ménagée par les éléments 23 et 24, n'est pas nécessairement cylindrique.

De façon à s'adapter à la forme et à la taille du drain, on peut prévoir des pièces, destinées à être placées sur la paroi interne des éléments 23 et 24, de façon à adapter la forme et la taille de la cavité au drain utilisé.

On peut également prévoir des moyens constitués en un matériau élastique et anti-dérapant, sur la paroi interne de la cavité 27, de façon à épouser la forme du drain 22.

Les dispositifs représentés en référence aux figures 1 à 5 sont réalisés en des matériaux utilisés classiquement pour un usage médical, tels que l'EVA ou le polyéthylène ou autres.

Enfin, grâce à la simplicité de sa conception, le dispositif est de fabrication peu coûteuse. Ceci présente de nombreux avantages en milieu hospitalier puisque ce dispositif peut être à usage unique.

Dans tous les exemples décrits, le dispositif selon l'invention est constitué de deux éléments car ceci correspond au mode de réalisation le plus simple, mais il pourrait en comprendre plus de deux.

Les signes de référence insérés après les caractéristiques techniques mentionnées dans les revendications, ont pour seul but de faciliter la compréhension de ces dernières, et n'en limitent aucunement la portée.

## Revendications

1. Dispositif de protection et de maintien de moyen de mise en communication (2, 22) de l'intérieur du corps d'un patient avec l'extérieur, ledit dispositif comportant les moyens de mise en communication et comprenant au moins deux éléments (3, 4; 23, 24) déterminant une position de fermeture dudit dispositif dans laquelle lesdits éléments sont solidaires et définissent une cavité (5, 21) pour le passage desdits moyens (2, 22), et une position d'ouverture dans laquelle lesdits éléments (3, 4; 23, 24) sont écartés l'un de l'autre pour ouvrir la cavité et permettre le retrait ou la mise en place latérals dudit dispositif sur lesdits moyens de mise en communication ledit dispositif comportant au moins une pièce en saillie (6, 25, 26), fixée auxdits éléments, ladite pièce en saillie formant un plan sensiblement perpendiculaire à l'axe de la cavité, les moyens de mise en communication étant des sondes ou des drains, caractérisé en ce que les éléments (3, 4; 23, 24) sont séparables et qu'ils comprennent des moyens (8, 31) permettant le coulissement d'un élément (4, 24) dans l'autre (3, 23), en ce que dans la position de fermeture, la cavité définie par lesdits éléments présente un diamètre intérieur correspondant sensiblement au diamètre extérieur desdits moyens pour assurer leur maintien, lesdits éléments (3, 4; 23, 24) recouvrant totalement la partie desdits moyens de mise en communication se trouvant à l'intérieur de la cavité (5, 27) et pouvant ainsi assurer à eux seuls leur maintien dans ledit dispositif.

2. Dispositif selon la revendication 1, caractérisé en ce qu'il est constitué de deux éléments (3, 4; 23, 24) sensiblement semi-cylindriques.

3. Dispositif selon la revendication 1, caractérisé en ce que les éléments (3, 4; 23, 24) définissent une cavité adaptée à la forme du moyen de mise en communication.

4. Dispositif selon l'une des revendications 1 à 3, caractérisé en ce que la paroi Interne de la cavité (5; 27) est recouverte d'un matériau élastique et anti-dérapant.

5. Dispositif selon l'une des revendications 1 à 4, dans lequel au moins une pièce (93, 94) en un matériau rigide est placée sur la paroi interne de la cavité (5, 27).

6. Dispositif selon l'une quelconque des revendications précédentes, caractérisé en ce que ladite pièce en saillie (6; 25, 26) est réalisée en un matériau rigide.

7. Dispositif selon l'une quelconque des revendications précédentes, caractérisé en ce que la pièce en saillie comprend une pièce (28) réalisée en une matière absorbante, de préférence fixée à une partie rigide (25, 26).

8. Dispositif selon l'une quelconque des revendications précédentes, caractérisé en ce qu'il comprend des moyens de fixation (7) sur ledit corps.

9. Dispositif selon l'une quelconque des revendications 1 à 7, caractérisé en ce qu'il comprend des moyens de fixation constitués par une zone adhésive placée sur la face arrière (29, 30).

10. Dispositif selon l'une quelconque des revendications précédentes dans lequel les parties au contact du corps sont recouvertes d'un matériau anti-escarre.

11. Dispositif selon l'une quelconque des revendications précédentes, caractérisé en ce qu'il est à usage unique.

## Claims

1. A device for protection and holding means of communications (2, 22) between the inside of a patient's body with the outside, whereas the said device comprises the means of communications and contains at least two elements (3, 4; 23, 24) delineating a closed position of the device in which the said elements are interlocked and define a cavity (5, 27) for the passage of the said means (2, 22) and an open position in which the said elements (3, 4; 23, 24) are at a distance from one another in order to open the cavity and enable lateral retraction and placement of the said device on the said means of communications, whereas the said device comprises at least one protruding part (6, 25, 26) attached to the said elements, whereby the protruding part forms a plane that is more or less perpendicular to the axis of the cavity, whereas the means of communications are probes or drains, characterised in that the elements (3, 4; 23, 24) are separable and in that they comprise means (8, 31) enabling one element (4, 24) to slide into the other (3, 23), in that in the closed position, the cavity delineated by the said elements exhibits an inside diameter corresponding more or less to the outside diameter of the said means to hold them, whereas the said elements (3, 4; 23, 24) cover totally the portion of the said means of communications located inside the cavity (5, 27) and can hold them inside the said device.

2. A device according to claim 1, characterised in that it consists of two elements (3, 4; 23, 24) more or less semi-cylindrical.

3. A device according to claim 1, characterised in that the elements (3, 4; 23, 24) delineate a cavity suited to the shape of the means of communications.

4. A device according to one of the claims 1 to 3, characterised in that the inside wall of the cavity (5; 27) is covered with an elastic and antiskid material.

5. A device according to one of the claims 1 to 4, in which at least one part (93, 94) made of a rigid material is placed on the inside wall of the cavity (5, 27).

6. A device according to any of the previous claims, characterised in that the said protruding part (6; 25, 26) is made of a rigid material.

7. A device according to any of the previous claims, characterised in that the said protruding part comprises a part (28) made of an absorbing material, preferably attached to a rigid part (25, 26).

8. A device according to any of the previous claims, characterised in that it comprises means for attachment (7) onto the said body.

9. A device according to any of the claims 1 to 7, characterised in that it comprises attachment means made of an adhesive zone placed on the rear face (29, 30).

10. A device according to any of the previous claims, in which the parts in contact with the body are covered with an eschar-preventing material.

11. A device according to any of the previous claims, characterised in that it is a single-use item.

## Patentansprüche

1. Vorrichtung zum Schutz und zur Halterung von Mitteln zur Verbindung (2, 22) des Inneren des Körpers eines Patienten mit dem Äußeren, wobei die Vorrichtung die Verbindungsmittel und mindestens zwei Elemente (3, 4; 23, 24) umfaßt, die eine Verschlussposition der Vorrichtung, in der die Elemente fest verbunden sind und einen Hohlraum (5, 27) für den Durchgang der Mittel (2, 22) definieren, und eine Öffnungsposition, in der die Elemente (3, 4; 23, 24) voneinander entfernt sind, um den Hohlraum zu öffnen und das seitliche Herausziehen oder die seitliche Anordnung der Vorrichtung auf den Verbindungsmitteln zu ermöglichen, bestimmen, wobei die Vorrichtung mindestens ein vorspringendes Teil (6, 25, 26) umfaßt, das an den Elementen befestigt ist, wobei das vorspringende Teil eine zur Achse des Hohlraumes im wesentlichen senkrechte Ebene bildet, wobei die Verbindungsmittel Sonden oder Drains sind, dadurch gekennzeichnet, dass die Elemente (3, 4; 23, 24) getrennt werden können und dass sie Mittel (8, 31) umfassen, die das Gleiten eines Elements (4, 24) in dem anderen (3, 23) ermöglichen, dass in der Verschlussposition der von den Elementen definierte Hohlraum einen Innendurchmesser aufweist, der im wesentlichen dem Außendurchmesser der Mittel entspricht, um deren Halt zu gewährleisten, wobei die Elemente (3, 4; 23, 24) zur Gänze den Teil der Verbindungsmittel abdecken, der sich im Inneren des Hohlraums (5, 27) befindet, und auf diese Weise selbst ihren Halt in der Vorrichtung gewährleisten können.

2. Vorrichtung nach Anspruch 1, dadurch gekennzeichnet, dass sie aus zwei im wesentlichen halbzylindrischen Elementen (3, 4; 23, 24) gebildet ist

3. Vorrichtung nach Anspruch 1, dadurch gekennzeichnet, dass die Elemente (3, 4; 23, 24) einen Hohlraum definieren, der an die Form des Verbindungsmittels angepasst ist.

4. Vorrichtung nach einem der Ansprüche 1 bis 3, dadurch gekennzeichnet, dass die Innenwand des Hohlraums (5; 27) von einem elastischen und rutschfesten Material bedeckt ist.

5. Vorrichtung nach einem der Ansprüche 1 bis 4, bei der mindestens ein Teil (93, 94) aus einem starren Material auf der Innenwand des Hohlraumes (5, 27) angeordnet ist.

6. Vorrichtung nach einem der vorstehenden Ansprüche, dadurch gekennzeichnet, dass das vorspringende Teil (6; 25, 26) aus einem starren Material besteht.

7. Vorrichtung nach einem der vorstehenden Ansprüche, dadurch gekennzeichnet, dass das vorspringende Teil ein Teil (28) umfaßt, das aus einem absorbierenden Material besteht und vorzugsweise auf dem starren Teil (25, 26) befestigt ist.

8. Vorrichtung nach einem der vorstehenden Ansprüche, dadurch gekennzeichnet, dass sie Mittel (7) zur Befestigung auf dem Körper des Patienten umfaßt.

9. Vorrichtung nach einem der Ansprüche 1 bis 7, dadurch gekennzeichnet, dass sie Befestigungsmittel umfaßt, die aus einer Klebezone gebildet sind, die auf der Rückseite (29, 30) vorgesehen ist.

10. Vorrichtung nach einem der vorstehenden Ansprüche, bei der die mit dem Körper des Patienten in Kontakt stehenden Teile mit einem Material zur Verhinderung der Schorfbildung überzogen sind.

11. Vorrichtung nach einem der vorhergehenden Ansprüche, dadurch gekennzeichnet, dass sie für einmalige Verwendung bestimmt ist.
